# EUROPÄISCHE PATENTANMELDUNG

(11) **EP 4 487 800 A1**
(43) Veröffentlichungstag der Anmeldung: **08.01.2025**
(21) Anmeldenummer: 23183656.0
(22) Anmeldetag: 05.07.2023
(51) Int. Cl.: A61B 18/18

(54) **MEDIZINISCHES SYSTEM ZUR UNTERSTÜTZUNG BEI EINER ABLATIONSTHERAPIE**

(71) Anmelder: Siemens Healthineers AG, 91301 Forchheim (DE)
(72) Erfinder: Kellnberger, Stephan, 91054 Erlangen (DE); Regensburger, Alois, 91099 Poxdorf (DE)
(74) Vertreter: Siemens Healthineers Patent Attorneys

(57) **Zusammenfassung**

Medizinisches System zur Unterstützung bei einer Ablationstherapie in einem Ablationsbereich, umfassend: mindestens eine nagelförmige Antenne mit einem entlang einer Längsachse bewegbaren leitenden Element, welche dazu eingerichtet ist, elektromagnetische Strahlungen in Mikrowellen- oder Radiofrequenzen abzustrahlen; eine Temperaturmessungseinheit, welche dazu ausgelegt ist, eine Temperatur zu messen und entsprechende Signale auszugeben; eine Simulationseinheit, welche dazu eingerichtet ist, basierend auf dem Standort der mindestens einen Antenne und der abgestrahlten elektromagnetischen Strahlungen und mit Hilfe eines Wärmeausbreitungsmodells, eine Simulation der Temperaturverteilung in dem Ablationsbereich bereitzustellen; eine Konfigurationseinheit, welche dazu eingerichtet ist, basierend auf den Signalen der Temperaturmessungseinheit und der bereitgestellten Simulation der Temperaturverteilung, eine Betriebskonfiguration der mindestens einen Antenne zu ermitteln, wobei die ermittelten Betriebskonfiguration mindestens eine Bewegung des leitenden Elements entlang der Längsachse der nagelförmigen Antenne umfassen; und eine Ausgabeeinheit, welche dazu eingerichtet ist, ein der ermittelten Betriebskonfiguration entsprechenden Konfigurationssignal auszugeben.

## Beschreibung

Die vorliegende Erfindung betrifft ein medizinisches System zur Unterstützung, beispielsweise eines Chirurgen, bei einer Ablationstherapie. Ferner bezieht sich die vorliegende Erfindung auf ein computerimplementiertes Verfahren zur Unterstützung bei einer Ablationstherapie.

Unabhängig vom grammatikalischen Geschlecht eines bestimmten Begriffes sind Personen mit männlicher, weiblicher oder anderer Geschlechteridentität mit umfasst.

Eine Ablation ist ein medizinisches Verfahren, damit Körpergewebe durch Hitze entfernt werden können. Ein Ablationseingriff oder eine Ablationstherapie kann verwendet werden, um Tumorgewebe zu entfernen aber auch um Läsionen eines Weichgewebes zu behandeln. Eine Ablationstherapie wird grundsätzlich an Organen wie Leber, Nieren, Lungen, Herzen oder Blasen durchgeführt.

Bei einer Ablation werden eine oder mehrere Nadeln verwendet, die perkutan in den Körper eines Patienten bis zu einem Ablationsbereich eingeführt werden. Die Nadeln umfassen eine oder mehrere Antennen, die elektromagnetische Strahlung in Mikrowellenfrequenzen oder Radiofrequenzen übertragen und durch eine distale Elektrode abstrahlen.

Die Geometrie einer Ablationsantenne ermöglicht, eine lokalisierte Behandlung bei einer Ablation durchführen zu können. Zur Behandlung größerer Tumoren oder zur Behandlung einer Vielzahl von örtlich verteilten Tumoren ist eine Ausbreitung der Wärme von Vorteil. Herkömmlicherweise wird hierbei eine Verteilung der abgestrahlten Wärme durch den Einsatz mehrerer Nadeln oder durch die Verwendung schirmförmiger Antennen erreicht.

Mit Hilfe dieser möglichen Anordnungen wird einen Ablationseingriff geplant. Es ist aktuell jedoch schwierig, einen Ablationseingriff planmäßig durchzuführen. Eine erhebliche Schwierigkeit besteht darin, dass herkömmliche Methoden nicht in der Lage sind, die erforderlichen Betriebsänderungen der Ablationsantennen zu bestimmen und sie in Echtzeit auszuführen, um die Ablationseingriff nach Plan zu folgen.

Das Dokument WO 91/16859 beispielsweise offenbart ein Gerät zur Erwärmung eines Gewebes eines Patienten mit Radiofrequenzen. Das Gerät umfasst einen Temperatursensor und eine wärmeleitende Elektrode, die nicht gleichzeitig tätig sein können. Um ein Temperatursignal zu empfangen muss die Elektrode untätig bleiben. Eine Steuerschaltung vergleicht das Temperatursignal mit einem Sollwert und dementsprechend moduliert die Leistung der Elektrode. Demnach muss die Elektrode wiederholt an- und ausgeschaltet werden.

Es ist daher eine Aufgabe der vorliegenden Erfindung, ein medizinisches System zu schaffen, die eine verbesserte, präzise und effiziente Bestimmung der erforderlichen Betriebsänderungen der Ablationsantennen bereitstellt, damit ein Ablationseingriff planmäßig durchgeführt werden kann.

Die Aufgabe der vorliegenden Erfindung wird durch ein System mit den Merkmalen des Anspruchs 1 und ein computerimplementiertes Verfahren mit den Merkmalen des Anspruchs 8 gelöst. Bevorzugte Ausführungsformen der Erfindung mit vorteilhaften Merkmalen sind in den abhängigen Ansprüchen angegeben.

Gemäß einer ersten Aspekt der Erfindung umfasst ein medizinisches System zur Unterstützung bei einer Ablationstherapie in einem Ablationsbereich mindestens eine nagelförmige Antenne mit einem entlang einer Längsachse bewegbaren leitenden Element, welche dazu eingerichtet ist, elektromagnetische Strahlungen im Mikrowellen- oder Radiofrequenzen abzustrahlen; eine Temperaturmessungseinheit, welche dazu ausgelegt ist, eine Temperatur zu messen und entsprechende Signale auszugeben; eine Simulationseinheit, welche dazu eingerichtet ist, basierend auf dem Standort der mindestens einen Antenne und der abgestrahlten elektromagnetischen Strahlungen und mit Hilfe eines Wärmeausbreitungsmodells, eine Simulation der Temperaturverteilung in dem Ablationsbereich bereitzustellen; eine Konfigurationseinheit, welche dazu eingerichtet ist, basierend auf den Signalen der Temperaturmessungseinheit und der bereitgestellten Simulation der Temperaturverteilung, eine Betriebskonfiguration der mindestens einen Antenne zu ermitteln, wobei die ermittelte Betriebskonfiguration mindestens eine Bewegung des leitenden Elements entlang der Längsachse der nagelförmigen Antenne umfassen; und eine Ausgabeeinheit, welche dazu eingerichtet ist, ein der ermittelten Betriebskonfiguration entsprechenden Konfigurationssignal auszugeben.

Bei einem Ablationsbereich kann es sich im Allgemeinen um alle Weichgewebe eines Patienten handeln, insbesondere um Organe wie Leber, Nieren, Lungen, Herzen oder Blasen, bei denen die Ablation zur Entfernung von Tumoren oder zur Behandlung von Läsionen verwendet wird.

In der vorliegenden Patentanmeldung ist eine Antenne als eine Mikrowellen- oder Radiofrequenzantenne, wie sie üblicherweise in Ablationstherapie benutzt werden, zu verstehen. Die Antenne ist an einem ersten Ende (das proximale Ende) mit einer Quelle elektromagnetischer Strahlung verbunden und ist an einem zweiten Ende (das distale Ende) mit einer Elektrode ausgestattet. Die Antenne umfasst ein leitendes Element (z.B. ein Hartmetallrohr), das die Ausbreitung elektromagnetischer Strahlungen ermöglicht. Die Antenne wird von einer Führungsrohr-Längenhaltestruktur (z.B. einem Nylonmantel) umhüllt, die dafür sorgt, dass die Antenne geschützt und gezielt geführt werden kann. Als solche "Nadel" kann die Antenne während der Ablationstherapie in den Körper eines Patienten perkutan eingeführt werden, mit der Elektrode in der Mitte des Ablationsbereichs (z.B. in der Mitte eines Tumors).

Eine Temperaturmessungseinheit kann jede Vorrichtung sein, die mit Sensoren zur Ermittlung der Temperatur innerhalb des Körpers eines Patienten und mit Mittel, um entsprechende Signalen auszugeben, ausgestattet ist. In Abhängigkeit davon, wo die Sensoren angebracht sind können die Temperatursensoren Kontakttemperatursensoren (darunter Thermoelemente, Thermistoren und Widerstandstemperaturdetektoren) umfassen oder aus berührungslosen Temperatursensoren (z.B. Pyrometer oder Strahlungsthermometer) bestehen. Die Übermittlung des Signals kann kabelgebunden oder kabellos erfolgen.

Ein Wärmeausbreitungsmodell ist eine mathematische Darstellung im Form einer Diffusionsgleichung, d.h. eine Differentialgleichung, welche die Ausbreitung der Temperatur mit Zeit an bestimmten Ortskoordinaten bei Vorhandensein örtlicher Wärmequelle ergibt. Ein solches Wärmeausbreitungsmodell kann beispielsweise auf Pennes Biowärmeübertragungsgleichung basieren.

Eine Betriebskonfiguration betrifft alle die Variablen und Parameter, die den Betrieb einer oder mehreren Antennen bestimmen. Diese Änderungsmöglichkeiten beinhalten beispielsweise den Abstand der Antenne zu dem Ablationsbereich, Eigenschaften der elektromagnetischen Strahlung, wie z.B. die Betriebsleistung oder die Betriebsfrequenz, oder die Anzahl und Standort der Antenne, wenn mehr als eine Antenne eingesetzt ist. Es ist vorgesehen, dass die Betriebskonfigurationen verschiedene Kombinationen der oben genannten Änderungsmöglichkeiten aufweisen, um verschiedene zusammenwirkende Effekte zu erzeugen. Beispielsweise kann eine Änderung der Eigenschaften der elektromagnetischen Strahlung in Kombination mit einer Bewegung des leitenden Elements entlang der Längsachse der nagelförmigen Antenne dazu führen, dass eine verbesserte Handhabung des reaktiven Nahfelds der Antenne ermöglicht wird.

Die verschiedenen Einheiten (Temperaturmessungseinheit, Simulationseinheit, Konfigurationseinheit und Ausgabeeinheit) umfassen im Allgemeinen Geräte und Computerprogramme, die Daten empfangen, verarbeiten und auswerten können. Die Einheiten sind kabelgebunden und/oder drahtlos miteinander verbunden, um Signale austauschen zu können. Daher umfassen die Einheiten mindestens eine zentrale Verarbeitungseinheit (CPU) und/oder mindestens eine Grafikverarbeitungseinheit (GPU) und/oder mindestens ein feldprogrammierbares Gate-Array (FPGA) und/oder mindestens eine anwendungsspezifische integrierte Schaltung (ASIC) und/oder eine beliebige Kombination der vorgenannten Elemente. Jedes Element kann ferner einen Arbeitsspeicher umfassen, der operativ mit der mindestens einen CPU verbunden ist, und/oder einen nicht-flüchtigen Speicher, der operativ mit der mindestens einen CPU und/oder dem Arbeitsspeicher verbunden ist. Jedes Element kann teilweise und/oder vollständig in einem lokalen Gerät und/oder teilweise und/oder vollständig in einem entfernten System, wie etwa einer Cloud-Computing-Plattform, implementiert sein.

Insbesondere die Simulationseinheit und die Konfigurationseinheit können eine Software, eine App oder einen Algorithmus mit unterschiedlichen Fähigkeiten zur Datenverarbeitung ausführen. Die Konfigurationseinheit kann vorteilhaft einen Algorithmus implementieren, der dazu eingerichtet ist, auf Basis der von den Sensoren gemessenen Temperaturwerten, eine Interpolation und/oder Extrapolation der Temperaturverteilung über den gesamten Ablationsbereich zu erstellen.

Es ist vorgesehen, dass einige der verwendeten Algorithmen vollständig oder teilweise von künstlichen Intelligenz unterstützt werden könnten.

Unter künstlicher Intelligenz ist hierbei eine computergestützte Einheit zu verstehen, die in der Lage ist, verschiedene Datenanalysemethoden zu implementieren, die im Allgemeinen als künstliche Intelligenz, maschinelles Lernen, Deep Learning oder Computerlernen bezeichnet werden.

In einigen Ausführungsformen der Erfindung ist ein künstliche Intelligenz-Modul vorgesehen. Das künstliche Intelligenz-Modul implementiert mindestens ein künstliches Intelligenz-Modell, bei dem es sich um ein mehrschichtiges Perzeptron-Netzwerk (MLP), ein rekurrentes neuronales Netzwerk (RNN), ein LSTM-Netzwerk (Long Short Memory), ein faltungsneuronales Netzwerk (CNN) oder ein anderes neuronales Netzwerk handeln kann.

Die Ausgabeeinheit kann insbesondere eine Schnittstelle, beispielsweise eine Application Programming Interface (API) umfassen, damit sie die von der Konfigurationseinheit ermittelte Betriebskonfiguration einlesen und das entsprechende Konfigurationssignal ausgeben könnte.

Die verschiedenen Einheiten können in Hardware und/oder Software, kabelgebunden und/oder drahtlos und in jeder Kombination davon realisiert sein. Sie können ferner eine Schnittstelle zu einem Intranet oder dem Internet, zu einem Cloud-Computing-Dienst, zu einem entfernten Server und/oder dergleichen umfassen.

In Systemen, die auf der Cloud-Computing-Technologie basieren, ist eine große Anzahl von Geräten über das Internet mit einem Cloud-Computing-System verbunden. Die Geräte können sich in einer Remote-Einrichtung befinden, die mit dem Cloud-Computing-System verbunden ist. Die Geräte können z.B. Geräten, Sensoren, Aktoren, Robotern und/oder Maschinen in einer oder mehreren Industrieanlagen umfassen oder daraus bestehen. Bei den Geräten kann es sich um Haushaltsgeräte oder Bürogeräte in einer Wohn-/Gewerbeeinrichtung handeln.

Das Cloud-Computing-System kann die Remote-Konfiguration, - Überwachung, -Steuerung und -Instandhaltung der angeschlossenen Geräte (auch allgemein als "Assets" bezeichnet) ermöglichen. Außerdem kann das Cloud-Computing-System die Speicherung großer Datenmengen, die regelmäßig von den Geräten erfasst werden, die Analyse der großen Datenmengen und die Bereitstellung von Erkenntnissen (z. B. Leistungsindikatoren, Ausreißer) und Warnungen für Bediener, Außendiensttechniker oder Eigentümer der Geräte über eine grafische Benutzerschnittstelle (z. B. von Webanwendungen) erleichtern. Die Erkenntnisse und Warnungen können die Steuerung und Instandhaltung der Geräte ermöglichen, was zu einem effizienten und ausfallsicheren Betrieb der Geräte führt. Das Cloud-Computing-System kann auch die Änderung von Parametern ermöglichen, die mit den Geräten verbunden sind, und Steuerbefehle, basierend auf der Grundlage der Erkenntnisse und Warnungen, über die grafische Benutzerschnittstelle ausgeben.

Das Cloud-Computing-System kann eine Vielzahl von Servern oder Prozessoren (auch als "Cloud-Infrastruktur" bezeichnet) umfassen, die geografisch verteilt und über ein Netzwerk miteinander verbunden sind. Auf den Servern/Prozessoren ist eine spezielle Plattform (im Folgenden als "Cloud-Computing-Plattform" bezeichnet) installiert, die die oben genannten Funktionen als Service (im Folgenden als "Cloud-Service" bezeichnet) bereitstellt. Die Cloud-Computing-Plattform kann eine Vielzahl von Softwareprogrammen umfassen, die auf einem oder mehreren Servern oder Prozessoren des Cloud-Computing-Systems ausgeführt werden, um die Bereitstellung des angeforderten Services für die Geräte und ihre Nutzer zu ermöglichen.

Eine oder mehrere APIs werden in dem Cloud-Computing-System eingesetzt, um den Nutzern verschiedene Cloud-Dienste anzubieten.

Nach einem zweiten Aspekt schafft die Erfindung ferner ein computerimplementiertes Verfahren zur Unterstützung bei einer Ablationstherapie in einem Ablationsbereich, mit den Schritten: (a) Bereitstellen, basierend auf dem Standort der mindestens einen Antenne und der abgestrahlten elektromagnetischen Strahlungen und mit Hilfe eines Wärmeausbreitungsmodells, einer Simulation der Temperaturverteilung in dem Ablationsbereich; (b) Messen einer Temperatur mit Hilfe einer Temperaturmessungseinheit; (c) Ermitteln, basierend auf der ermittelten Temperatur und der bereitgestellten Simulation der Temperaturverteilung in dem Ablationsbereich, einer Betriebskonfiguration der mindestens einen Antenne, wobei die ermittelte Betriebskonfiguration mindestens eine Bewegung des leitenden Elements entlang der Längsachse der nagelförmigen Antenne umfasst; und (d) Ausgeben eines der ermittelten Betriebskonfiguration entsprechenden Konfigurationssignals.

Das computerimplementiertes Verfahren gemäß dem zweiten Aspekt der Erfindung kann mit dem System gemäß dem ersten Aspekt der Erfindung durchgeführt werden. Die hierin im Zusammenhang mit dem System beschriebenen Merkmale und Vorteile sind daher auch für das Verfahren anwendbar und umgekehrt.

Gemäß einem dritten Aspekt schafft die Erfindung ferner ein Computerprogrammprodukt, das einen ausführbaren Programmcode umfasst, der dazu eingerichtet ist, bei seiner Ausführung das Verfahren gemäß dem zweiten Aspekt der vorliegenden Erfindung durchzuführen.

Gemäß einem vierten Aspekt stellt die Erfindung ein nicht-flüchtiges computerlesbares Datenspeichermedium bereit, das einen ausführbaren Programmcode umfasst, der dazu eingerichtet ist, bei seiner Ausführung das Verfahren gemäß dem zweiten Aspekt der vorliegenden Erfindung durchzuführen.

Das nicht-flüchtige, computerlesbare Datenspeichermedium kann jede Art von Computerspeicher, insbesondere einen Halbleiterspeicher, wie z. B. einen Festkörperspeicher umfassen oder daraus bestehen. Das Datenspeichermedium kann auch eine CD, eine DVD, eine Blu-Ray-Disc, einen USB-Speicherstick oder dergleichen umfassen oder daraus bestehen.

Gemäß einem fünften Aspekt schafft die Erfindung einen Datenstrom, der einen ausführbaren Programmcode umfasst oder dazu eingerichtet ist, einen solchen zu erzeugen, und der dazu eingerichtet ist, bei seiner Ausführung das Verfahren gemäß dem zweiten Aspekt der vorliegenden Erfindung durchzuführen.

Eine der Erfindung zugrundeliegende Idee besteht darin, ein System zur Unterstützung bei einer Ablationstherapie zu schaffen, das Abweichungen von einem vorgeplanten Ablauf eines Ablationseingriffs während des Ablationseingriffs schnell und effizient korrigieren kann. Das System umfasst eine oder mehrere Antennen, die elektromagnetischen Wellen in den Mikrowellen- oder Radiofrequenzen abstrahlen können und eine Simulationseinheit, welche eine dem geplanten Ablationseingriff entsprechenden Simulation der Temperaturverteilung erzeugt. Darüber hinaus ist eine Temperatureinheit, die gemessenen Temperaturwerte des Ablationsbereichs ermitteln. Diese Temperaturwerte sind von einer Konfigurationseinheit verarbeitet und mit der Simulation verglichen. Durch ermittelte Anpassungen der Betriebsbedingungen der Antenne sorgt das System dafür, dass Abweichungen von dem planmäßigen Ablauf des Ablationseingriffs korrigiert werden könnten.

Das vorstehend beschriebenes System ermöglicht vorteilhaft eine Implementierung eines computerimplementiertes Verfahrens zur Unterstützung bei einer Ablationstherapie. Hierbei wird zunächst (d.h. vor Anfang des Ablationseingriffs) eine Simulation der Temperaturverteilung in dem Ablationsbereich anhand eines Wärmeausbreitungsmodells bereitgestellt, die ein planmäßiger Ablauf des Ablationseingriffs darstellt. Daraufhin wird die Temperatur an verschiedenen Stellen des Ablationsbereichs durch den Einsatz von Temperatursensoren überwacht. Basierend auf Abweichungen zwischen den tatsächlichen und den simulierten Temperaturwerten wird eine Betriebskonfiguration ermittelt, wonach die Abweichungen korrigiert werden. Zuletzt wird die ermittelte Betriebskonfiguration durch ein entsprechendes Konfigurationssignal ausgegeben, damit die erforderlichen Änderungen der Betriebskonfiguration von einem Nutzer oder automatisch ausgeführt werden könnten.

Ein Vorteil der vorliegenden Erfindung ist, dass mit Hilfe der verschiedenen möglichen Parameteränderungen der einen oder mehreren Antennen kann das System effizient und flexibel an die verschiedenen erforderlichen Betriebskonfigurationen angepasst werden. Dadurch kann eine Ablationstherapie planmäßig erfolgen.

Ein weiterer Vorteil der Erfindung besteht darin, dass die Betriebskonfigurationen innerhalb von Sekunden umgesetzt werden können. Dies ermöglicht insbesondere, eine dynamische Anpassung oder Aktualisierung der elektromagnetischen Felder, damit die Ablationstherapie so reibungslos wie möglich laufen kann.

Vorteilhafte Ausführungsformen und Weiterentwicklungen ergeben sich aus den abhängigen Ansprüchen sowie aus der Beschreibung der verschiedenen bevorzugten Ausführungsformen, die in den beigefügten Figuren dargestellt sind.

Gemäß manchen Ausführungsformen der Erfindung ist vorgesehen, dass die Betriebskonfiguration ferner umfasst: eine Umstellung der Leistung der elektromagnetischen Strahlungen; und/oder eine Umstellung der Frequenz der elektromagnetischen Strahlungen; und/oder eine Umstellung der Phase der elektromagnetischen Strahlungen. Damit kann man die wesentlichen Eigenschaften der elektromagnetischen Strahlungen in beliebiger Kombination ändern.

Gemäß manchen Ausführungsformen der Erfindung ist vorgesehen, dass das System mindestens zwei Antennen umfasst, wobei die erste Antenne mit einem ersten elektromagnetischen Feld und die zweite Antenne mit einem zweiten elektromagnetischen Feld versorgt sind, wobei das erste Feld als Hintergrundfeld dient. Eine solche Zusammenwirkung zweier Antennen sorgt dafür, dass der Ablationsbereich oder ein Teil davon mit Hilfe der ersten Antenne auf einer Basistemperatur gehalten werden kann. Die zweite Antenne kann dann verwendet werden, um extra Wärme lokal auszuüben. Die zweite Antenne kann vorteilhaft mit einem bewegbaren leitenden Element ausgestattet werden, um eine präzisere Arbeit leisten zu können. Durch diese konstruktive Überlagerung zweier elektromagnetischen Feldern kann ein präziseres und effizienteres Ablationsverfahren ablaufen.

Weitere Antennen können auch eingesetzt werden. Einige davon könnten zu dem Hintergrundfeld beitragen, während die anderen Antenne lokale Wärme liefern könnten.

Gemäß manchen Ausführungsformen der Erfindung ist vorgesehen, dass das System mindestens zwei Antennen umfasst, wobei die jeweiligen Phasen der abgestrahlten elektromagnetischen Strahlungen umstellbar sind. In etlichen vorteilhaften Ausführungsformen sind die Frequenzen der elektromagnetischen Strahlungen gleich. Dadurch kann eine Änderung der Phasen dazu führen, dass eine konstruktive bzw. destruktive Interferenz erzeugt werden kann. Dementsprechend kann die Wärme an verschiedenen Orten innerhalb des Ablationsbereiches geändert werden, ohne dass die Leistung der elektromagnetischen Strahlungen angepasst werden muss.

Gemäß manchen Ausführungsformen der Erfindung ist vorgesehen, dass das System mindestens zwei Antennen umfasst, wobei die Antennen innerhalb einer Führungsrohr-Längenhaltestruktur schirmförmig angeordnet sind, und wobei die Antennen elektromagnetische Strahlungen unterschiedlicher Eigenschaften abstrahlen. Hierbei können die oben angeführten Ausführungsformen bezüglich zwei oder mehr Antennen angewandt und kombiniert werden. Diese Ausführungsformen und Anordnungen der Antennen sind insbesondere vorteilhaft, wenn das System der Erfindung mit einem Endoskop verwendet wird.

Gemäß manchen Ausführungsformen der Erfindung ist vorgesehen, dass die Konfigurationssignale in einem zeitlichen Abstand von zumindest fünf Sekunden zueinander, vorzugsweise im Sekundentakt, ausgegeben werden. Um die Betriebskonfigurationen effektiv einsetzen zu können sollten die Konfigurationssignale innerhalb einer angemessene Zeitspanne ausgesandt werden, dass für die Unterstützung eines Chirurgen während einer Ablationstherapie nutzbar sein sollten. Die Konfigurationssignale können in lesbarer Form (z.B. auf einem Bildschirm) angezeigt werden. Die erforderlichen Anpassungen, um die Betriebskonfiguration einzusetzen (Änderungen der Leistung und/oder Frequenz und/oder Phase der elektromagnetischen Strahlungen und/oder Bewegung des leitenden Elements der Antenne) könnten entweder von dem Chirurgen oder automatisch vorgenommen werden.

Gemäß manchen Ausführungsformen der Erfindung ist vorgesehen, dass die Temperaturmessungseinheit eine Vielzahl von Temperatursensoren umfasst, welche in die mindestens eine Antenne integriert sind und/oder sich als gesonderten Messsonden auf dem Ablationsbereich befinden. Um die Temperatur auf dem Ablationsbereich umfangreich zu ermitteln ist es vorgesehen, dass die Temperatureinheit mit verschiedenen Temperatursensoren ausgestattet wird. Die in die Antennen integrierten Temperatursensoren sorgen dafür, dass die Temperatur am Ablationsort kontinuierlich ermittelt werden kann. Solche Sensoren könnten beispielsweise an der Spitze der Antenne und/oder daneben an verschiedenen Stellen des Körpers der Antenne angebracht werden. Diese Sensoren könnten vorteilhaft berührungslosen Temperatursensoren (z.B. Pyrometer oder Strahlungsthermometer) umfassen, welche die Temperaturwerte kabellos oder kabelgebunden übertragen könnten.

Die Temperatursensoren könnten ebenfalls an verschiedenen Stellen des Ablationsbereichs des Patienten verteilt werden, um die Temperatur an bestimmten Stellen im Lauf der Zeit zu übertragen. Diese Temperatursensoren können beispielsweise an der Spitze einer Nadel angeordnet sein, die perkutan bis zum Ablationsbereich eingeführt wird. Hierbei könnten Kontakttemperatursensoren (darunter Thermoelemente, Thermistoren und Widerstandstemperaturdetektoren) eingesetzt werden, welche die Temperaturwerte kabellos oder kabelgebunden übertragen könnten.

Obwohl hier und auch im Folgenden einige Funktionen als von Einheiten ausgeführt beschrieben werden, bedeutet dies nicht zwangsläufig, dass diese Einheiten als voneinander getrennte Einheiten bereitgestellt werden. In Fällen, in denen eine oder mehrere Einheiten oder auch ein Teil davon als Software bereitgestellt werden, können die Einheiten durch Programmcodeabschnitte oder Programmcodesegmente implementiert werden, die voneinander getrennt sein können, aber auch miteinander verwoben oder integriert sein können.

Ebenso können in Fällen, in denen eine oder mehrere Einheiten als Hardware bereitgestellt werden, die Funktionen einer oder mehrerer Einheiten durch ein und dieselbe Hardwarekomponente bereitgestellt werden oder die Funktionen mehrerer Einheiten können auf mehrere Hardwarekomponenten verteilt sein, die nicht notwendigerweise den Einheiten entsprechen müssen. Es ist daher davon auszugehen, dass jede Anwendung, jedes System, jedes Verfahren usw., das alle einer bestimmten Einheit zugeschriebenen Merkmale und Funktionen aufweist, diese Einheit umfasst oder implementiert. Insbesondere ist es möglich, dass alle Einheiten durch Programmcode implementiert werden, der von z.B. einem Server oder einer Cloud-Computing-Plattform, ausgeführt wird.

Alle genannten Ausführungsformen und Implementierungen können beliebig miteinander kombiniert werden, soweit dies sinnvoll ist.

Der weitere Umfang der Anwendbarkeit des vorliegenden Verfahrens und der Vorrichtung wird aus den folgenden Figuren, der detaillierten Beschreibung und den Ansprüchen ersichtlich werden. Es versteht sich jedoch, dass die detaillierte Beschreibung und die spezifischen Beispiele, zwar bevorzugte Ausführungsformen der Erfindung angeben, jedoch in erste Linie zur Veranschaulichung dienen und verschiedene Änderungen und Modifikationen innerhalb des grundlegenden Gedankens und des Umfangs der Erfindung für Fachleute erkennbar sind.

Nachstehend wird die Erfindung mit Bezug auf ihre vorteilhaften Ausführungsformen anhand der folgenden Zeichnungen beschrieben. Sie dienen dazu, Ausführungsformen von Konzepten, die die beanspruchte Erfindung beinhalten, weiter zu veranschaulichen und verschiedene Prinzipien und Vorteile dieser Ausführungsformen zu erläutern.

Es zeigen:
Fig. 1 ein medizinisches System zur Unterstützung bei einer Ablationstherapie nach einer Ausführungsform der Erfindung;
Fig. 2 ein medizinisches System zur Unterstützung bei einer Ablationstherapie nach einer weiteren Ausführungsform der Erfindung;
Fig. 3 ein schematisches Ablaufdiagramm zur Darstellung des Ablaufs eines computerimplementierten Verfahrens zur Unterstützung bei einer Ablationstherapie gemäß einer Ausführungsform der Erfindung;
Fig. 4 ein schematisches Blockdiagramm, das ein Computerprogrammprodukt gemäß einer Ausführungsform des dritten Aspekts der vorliegenden Erfindung darstellt; und
Fig. 5 ein schematisches Blockdiagramm, das ein nicht flüchtiges computerlesbares Datenspeichermedium gemäß einer Ausführungsform des vierten Aspekts der vorliegenden Erfindung zeigt.

Teile der verschiedenen Zeichnungen, die identische oder funktionell ähnliche Elemente in den einzelnen Ansichten darstellen, werden mit den gleichen Bezugszeichen bezeichnet.

Elementen, die in den Zeichnungen geschildert sind, sind nicht unbedingt maßgetreu dargestellt. Dies dient dazu, die Grundlagen und Prinzipien der Erfindung klar und deutlich zu offenbaren. In ähnlicher Weise werden in einigen Fällen bekannte Strukturen und Geräte in Form von Blockdiagrammen dargestellt, um mögliche Konzepte der vorliegenden Erfindung zu illustrieren.

Die Nummerierung der Schritte in den Verfahren soll deren Beschreibung erleichtern. Sie implizieren nicht unbedingt eine bestimmte Reihenfolge der Schritte. Insbesondere können mehrere Schritte gleichzeitig durchgeführt werden.

Die ausführliche Beschreibung der anliegenden Zeichnungen enthält spezifische Details, um ein umfassendes Verständnis der vorliegenden Erfindung zu ermöglichen. Dem Fachmann wird jedoch klar sein, dass die vorliegende Erfindung auch ohne diese spezifischen Details ausgeführt werden kann.

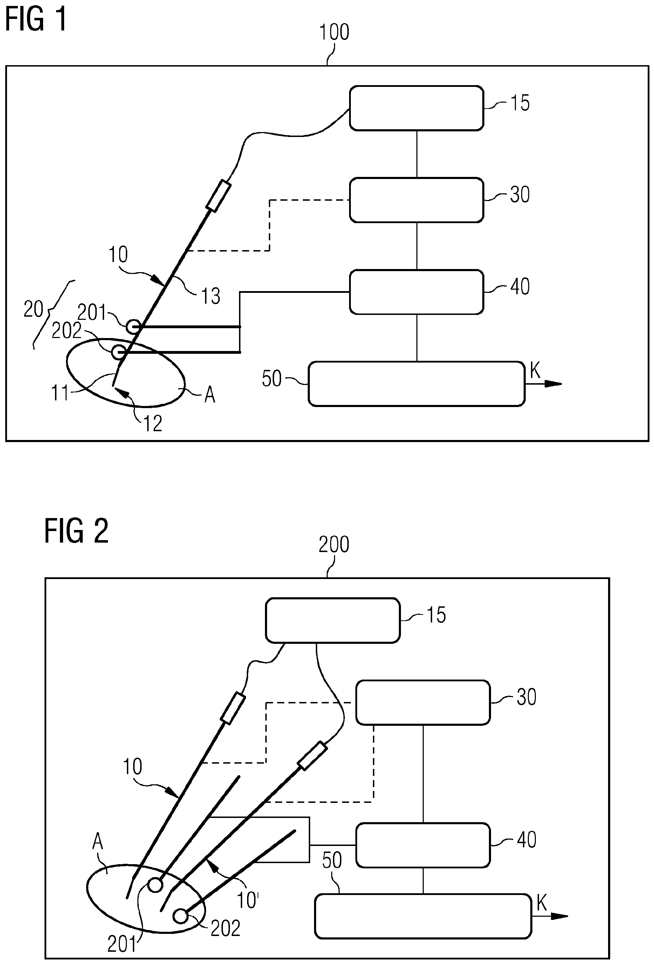

Fig. 1 zeigt ein medizinisches System 100 zur Unterstützung bei einer Ablationstherapie in einem Ablationsbereich A gemäß einer Ausführungsform der Erfindung.

Das im Fig. 1 veranschaulichte System 100 umfasst eine nagelförmige Antenne 10, eine Temperaturmessungseinheit 20, eine Simulationseinheit 30, eine Konfigurationseinheit 40 und eine Ausgabeeinheit 50.

Die nagelförmige Antenne 10 kann eine herkömmliche Ablationsantenne sein. Die Antenne 10 wird in der Regel von einer Führungsrohr-Längenhaltestruktur 13 (z.B. einem Nylonmantel) umhüllt, die dafür sorgt, dass die Antenne geschützt und gezielt geführt werden kann. Die Antenne 10 ist derart nagelförmig gestaltet, dass im Körper eines Patienten perkutan bis zu einem Ablationsbereich A eingeführt werden kann. Die Antenne 10 umfasst ein entlang ihrer Längsachse bewegbares leitendes Element 11, die dazu dient, elektromagnetische Strahlungen zu übertragen. Ein Ende des leitenden Elements 11 weist eine Elektrode 12 auf, welche die Spitze der Antenne 10 definiert. Während einer Ablationstherapie ist die Elektrode 12 in der Mitte des Ablationsbereichs A (z.B. in der Mitte eines Tumors) positioniert werden.

Der Ablationsbereich A kann im Allgemeinen einen Teil eines Weichgewebes eines Patienten sein, bei dem eine Ablationstherapie zur Entfernung von Tumoren oder zur Behandlung von Läsionen verwendet wird. Der Ablationsbereich A kann insbesondere Teil von Organen wie Leber, Nieren, Lungen Herzen oder Blasen sein.

Die Antenne 10 ist mit einer elektromagnetischen Strahlungsversorgungseinheit 15 verbunden. Die elektromagnetische Strahlungsversorgungseinheit 15 kann einen Generator sein, der dazu eingerichtet ist, elektromagnetische Strahlungen im Mikrowellen- oder Radiofrequenzen zu erzeugen. Die elektromagnetischen Strahlungen sind durch das leitende Element 11 der Antenne 10 übertragen und durch die Elektrode 12 abgestrahlt.

Die Antenne 10 kann von einem Chirurgen oder einem chirurgischen Roboter betätigt werden.

Die Temperaturmessungseinheit 20 ist dazu ausgelegt, eine Temperatur zu messen und entsprechende Signale auszugeben. Die Temperaturmessungseinheit 20 kann es sich um ein beliebiges Gerät oder Vorrichtung handeln, die in der Lage ist, die Temperatur im Körper eines Patienten mit Hilfe von Temperatursensoren 201, 202 zu messen, und ist mit Mittel zur Ausgabe entsprechender Signale ausgestattet. Die Temperaturmessungseinheit 20 kann ausschließlich aus Temperatursensoren 201, 202 bestehen.

Um eine umfangreiche Überwachung der Temperatur des Ablationsbereichs A zu ermöglichen sind erfindungsgemäß mehrere Temperatursensoren vorgesehen. Der Einfachheit halber sind in Fig. 1 nur zwei Temperatursensoren 201, 202 dargestellt, die an dem Körper der Antenne 10 angebracht sind. Die in die Antenne 10 integrierten Temperatursensoren 201, 202 sorgen dafür, dass die Temperatur am Ablationsort (d.h. die Stelle, wo die Elektrode 20 mit dem Ablationsbereich A in Kontakt steht) kontinuierlich ermittelt werden könnte. Andere Temperatursensoren könnten beispielsweise an der Spitze der Antenne 10 angebracht werden.

Die Temperatursensoren 201, 202 könnten berührungslosen Temperatursensoren (z.B. Pyrometer oder Strahlungsthermometer) und/oder Kontakttemperatursensoren (darunter Thermoelemente, Thermistoren und Widerstandstemperaturdetektoren) umfassen, welche die Temperaturwerte kabellos oder kabelgebunden übertragen könnten.

Die Simulationseinheit 30 ist dazu eingerichtet, eine Simulation der Temperaturverteilung in dem Ablationsbereich im Lauf der Zeit bereitzustellen. Die Simulation wird auf Basis des Standorts der mindestens einen Antenne 10 und der Eigenschaften der elektromagnetischen Strahlungen durchgeführt. Diese Information wird als Eingabe eines Wärmeausbreitungsmodells, das die Ausbreitung einer anfänglichen, durch die örtliche Verteilung der Antenne 10 und die Eigenschaften der elektromagnetischen Felder definierten Wärmeverteilung im Lauf der Zeit feststellt.

Das Wärmeausbreitungsmodell kann mit herkömmlichen Lösungsalgorithmen für Differentialgleichungen gelöst werden. Diese Algorithmen können von auf künstlicher Intelligenz basierten Methoden unterstützt. Dementsprechend umfasst in einigen Ausführungsformen der Erfindung die Simulationseinheit 30 ein künstliches Intelligenz-Modul und ein künstliches Intelligenz-Model, z.B. ein mehrschichtiges Perzeptron-Netzwerk (MLP), ein rekurrentes neuronales Netzwerk (RNN), ein LSTM-Netzwerk (Long Short Memory), ein faltungsneuronales Netzwerk (CNN) oder ein anderes neuronales Netzwerk.

Die Simulationseinheit 30 umfasst mindestens eine CPU und eine Schnittstelle mit einem Display, durch welche die Simulation (z.B. die Entwicklung der Isothermen auf dem Ablationsbereich A mit der Zeit) visualisierbar sein könnte.

Die Konfigurationseinheit 40 ist dazu eingerichtet, eine Betriebskonfiguration der mindestens einen Antenne 10 zu ermitteln, um die geplanten Ablationstherapie planmäßig folgen zu können. Die Konfigurationseinheit 40 erstellt eine Betriebskonfiguration durch Vergleich der tatsächlichen Temperaturverteilung des Ablationsbereichs A, die von den Daten der Temperatursensoren 201, 202 der Temperaturmessungseinheit 20 stammen, mit der von der Simulationseinheit 30 bereitgestellten Simulation der Temperaturverteilung. Die ermittelte Betriebskonfiguration dient dazu, Abweichungen von der geplanten Ablationstherapie zu korrigieren. Dies erfolgt durch die Anpassung von Betriebsparametern, z.B. durch Änderungen der Leistung und/oder Frequenz und/oder Phase der elektromagnetischen Strahlungen und/oder durch die Bewegung des leitenden Elements 11 der Antenne 10.

Die Konfigurationseinheit 40 kann mindestens eine CPU, einen Datenspeicher und eine Schnittstelle, beispielweise eine Application Programming Interface (API), umfassen. Die API kann mit dem Temperatureinheit 20 kabellos und/oder mit Kabel verbunden sein. Die CPU verfügt mindestens über eine Software, eine App oder einen Algorithmus mit unterschiedlichen Fähigkeiten zur Datenverarbeitung.

In Anbetracht dessen, dass die Temperatureinheit 20 nur Temperaturwerte von bestimmten Stellen übertragen kann ist es vorgesehen, dass die Konfigurationseinheit 40 insbesondere einen Algorithmus implementierte, der dazu eingerichtet ist, auf Basis der von den Temperatursensoren 201, 202 gemessenen Temperaturwerten, eine Interpolation und/oder Extrapolation der Temperaturverteilung über den gesamten Ablationsbereich A zu erstellen.

Die Ausgabeeinheit 50 ist zur Ausgabe eines der ermittelten Betriebskonfiguration entsprechenden Konfigurationssignals K ausgelegt. Die Ausgabeeinheit 50 kann mindestens eine CPU, einen Datenspeicher und eine Schnittstelle, beispielweise eine Application Programming Interface (API), umfassen. Die API kann mit der Konfigurationseinheit 40 verbunden sein, um die ermittelte Betriebskonfiguration zu empfangen. Die API kann auch mit einer Schnittstelle verbunden sein, um das Konfigurationssignal K lesbar für einen Nutzer (z.B. einen Chirurgen) zur Verfügung zu stellen. Das Konfigurationssignal K kann auch mit Antriebselementen verbunden sein, welche die Betriebskonfiguration ausführen können. Dies ist besonders vorteilhaft, wenn die geplante Ablation mit Hilfe eines chirurgischen Roboters durchzuführen ist. Die Verbindungen der Ausgabeeinheit 50 können kabellos und/oder mit Kabel erfolgen.

Im Ablauf einer Ablationstherapie sind eine Vielzahl von Konfigurationssignale K zu erwarten. Um die Betriebskonfigurationen effektiv einsetzten zu können sollten die Konfigurationssignale K innerhalb einer derart angemessenen Zeitspanne ausgesandt werden, dass für die Unterstützung eines Chirurgen während einer Ablationstherapie nutzbar sein sollten. In einigen vorteilhaften Ausführungsformen der Erfindung sind diese Konfigurationssignale K in einem zeitlichen Abstand von zumindest fünf Sekunden zueinander, vorzugsweise im Sekundentakt, ausgegeben werden. Die Konfigurationssignale K könnten in lesbarer Form (z.B. auf einem Bildschirm) angezeigt werden.

Fig. 2 zeigt eine weitere Ausführungsform eines erfindungsgemäßen medizinischen Systems 100 zur Unterstützung bei einer Ablationstherapie in einem Ablationsbereich A.

Die in Fig. 2 dargestellte Ausführungsform betrifft ein System 200 mit zwei Antennen 10, 10'. Jede Antenne 10, 10' könnte sein, wie die in Fig. 1 dargestellte Antenne 10. Die Antennen 10, 10'sind mit einem elektromagnetischen Strahlungsversorgungseinheit 15 kabelgebunden.

Die elektromagnetischen Strahlungsversorgungseinheit 15 sorgt dafür, dass die Antennen 10, 10' jeweils mit elektromagnetischen Strahlungen versorgt werden.

Gemäß einigen Ausführungsformen sind die erste Antenne 10 mit einem ersten elektromagnetischen Feld und die zweite Antenne 10' mit einem zweiten elektromagnetischen Feld versorgt werden. Der Ablationsbereich A oder ein Teil davon kann mit Hilfe der ersten Antenne 10 auf einer Basistemperatur gehalten werden. Die zweite Antenne 10' kann dann verwendet werden, um extra Wärme lokal auszuüben. Eine solche Zusammenwirkung zweier Antennen 10, 10' sorgt dafür, dass die erforderliche Temperatur erreicht werden kann, ohne dass die zweite Antenne 10' viel Wärme dafür verwenden muss. Dies führt zu einer schnellen und effizienten Ablauf der Ablationstherapie. Somit kann durch konstruktive Überlagerung zweier elektromagnetischen Feldern ein präziseres und effizienteres Ablationsverfahren ablaufen.

Gemäß einigen anderen Ausführungsformen sind die Frequenzen der elektromagnetischen Strahlungen der beiden Antennen 10, 10' gleich. Dadurch kann eine Änderung der jeweiligen Phasen dazu führen, dass eine konstruktive bzw. destruktive Interferenz erzeugt werden kann. Dementsprechend kann die Wärme an verschiedenen Orten innerhalb des Ablationsbereichs A geändert werden, ohne dass die Leistung der elektromagnetischen Strahlungen angepasst werden muss.

In Fig. 2 sind Temperatursensoren 201, 202 dargestellt. Im Gegensatz zu der in Fig. 1 gezeigten Anordnung sind die Temperatursensoren 201, 202 an verschiedenen Stellen des Ablationsbereichs A verteilt werden. Damit kann die Temperatur an bestimmten Stellen des Ablationsbereichs A im Lauf der Zeit überwacht. Die Temperatursensoren 201, 202 befinden sich an der Spitze einer jeweiligen Nadel, die perkutan bis zum Ablationsbereich A eingeführt sind. Hierbei könnten Kontakttemperatursensoren (darunter Thermoelemente, Thermistoren und Widerstandstemperaturdetektoren) eingesetzt werden, welche die Temperaturwerte kabellos oder kabelgebunden an die Konfigurationseinheit 40 übertragen könnten.

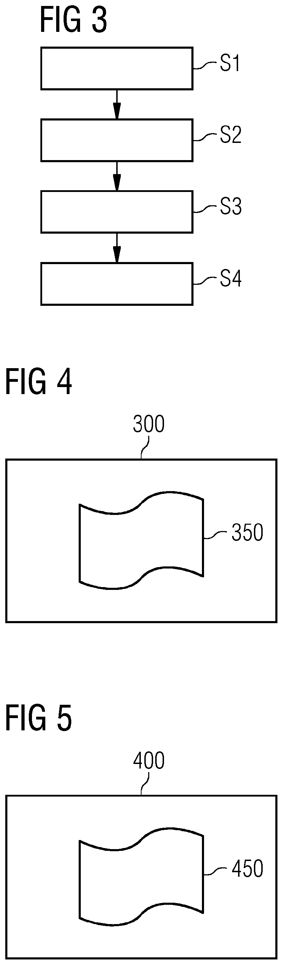

Fig. 3 zeigt in einem schematischen Ablaufdiagramm eine Darstellung des Ablaufs eines computerimplementierten Verfahrens zur Unterstützung bei einer Ablationstherapie nach einer Ausführungsform der Erfindung. Das Verfahren kann vorteilhaft mit den in Fig. 1 und in Fig. 2 dargestellten Systemen 100, 200 durchgeführt werden.

In einem Schritt S1 wird eine Simulation der Temperaturverteilung auf dem Ablationsbereich A bereitgestellt. Die Simulation erfolgt mit dem Einsatz eines Wärmeausbreitungsmodells, das die örtliche Verteilung der planmäßig eingesetzten Antennen 10, 10' und der Eigenschaften der jeweiligen elektromagnetischen Strahlungen, mit denen die Antennen 10, 10'versorgt werden, als Eingabe verwendet. Die Simulation entspricht den Ablauf einer geplanten Ablationstherapie.

In einem Schritt S2 wird die Temperatur an verschiedenen Stellen des Ablationsbereichs A durch den Einsatz von Temperatursensoren 201, 202 gemessen. Wie in Fig. 1 und 2 gezeigt könnten die Temperatursensoren 201, 202 an der Antenne 10 angebracht werden und/oder an verschiedenen Stellen des Ablationsbereichs A verteilt werden.

In einem weiteren Schritt S3 wird eine Betriebskonfiguration ermittelt. Die Betriebskonfiguration basiert auf einem Vergleich der tatsächlichen Temperaturverteilung des Ablationsbereichs A, die von den Daten der Temperatursensoren 201, 202 der Temperaturmessungseinheit 20 stammen, mit der bereitgestellten Simulation der Temperaturverteilung.

Die ermittelte Betriebskonfiguration ist derart ausgelegt, um Abweichungen von der geplanten Ablationstherapie, die durch die Simulation dargestellt wurde, zu korrigieren. Dies erfolgt durch die Anpassung von Betriebsparametern, wie beispielsweise die Leistung, die Frequenz oder die Phase der elektromagnetischen Strahlungen und/oder die Bewegung des leitenden Elements 11 der Antenne 10.

In einem Schritt S4 wird ein Konfigurationssignal K ausgegeben, die Information über die ermittelte Betriebskonfiguration enthält.

Im Ablauf einer Ablationstherapie werden die Schritte S2, S3 und S4 mehrmals wiederholt. Nach einigen bevorzugten Ausführungsformen der Erfindung sind die Konfigurationssignale K im Abstände von weniger als fünf Sekunden voneinander gesandt werden. Vorzugsweise wird jede Sekunde ein Konfigurationssignal K ausgegeben.

Fig. 4 zeigt ein schematisches Blockdiagramm, das ein Computerprogrammprodukt 300 gemäß einer Ausführungsform des dritten Aspekts der vorliegenden Erfindung illustriert. Das Computerprogrammprodukt 300 umfasst einen ausführbaren Programmcode 350, der so konfiguriert ist, dass er bei seiner Ausführung das Verfahren gemäß einer beliebigen Ausführungsform des zweiten Aspekts der vorliegenden Erfindung durchführt, insbesondere wie es in den vorangegangenen Figuren beschrieben wurde.

Fig. 5 zeigt ein schematisches Blockdiagramm, das ein nicht flüchtiges computerlesbares Datenspeichermedium 400 gemäß einer Ausführungsform des vierten Aspekts der vorliegenden Erfindung illustriert. Das Datenspeichermedium 400 umfasst einen ausführbaren Programmcode 450, der so konfiguriert ist, dass er, wenn er ausgeführt wird, das Verfahren gemäß einer beliebigen Ausführungsform des zweiten Aspekts der vorliegenden Erfindung durchführt, insbesondere wie es in den vorangegangenen Figuren beschrieben wurde.

Das nicht-flüchtige, computerlesbare Datenspeichermedium kann jede Art von Computerspeicher, insbesondere einen Halbleiterspeicher wie einen Festkörperspeicher, umfassen oder daraus bestehen. Das Datenspeichermedium kann auch eine CD, eine DVD, eine Blu-Ray-Disc, einen USB-Speicherstick oder ähnliches umfassen oder aus solchen bestehen.

Die vorstehende Beschreibung der offengelegten Ausführungsformen sind lediglich Beispiele für mögliche Ausführungsformen, die dem Fachmann die Möglichkeit geben sollen, die vorliegende Erfindung herzustellen oder zu verwenden. Verschiedene Variationen und Änderungen dieser Ausführungsformen werden für den Fachmann leicht ersichtlich sein, und die hierin definierten allgemeinen Grundsätze können auf andere Ausführungsformen angewandt werden, ohne dass der Kern oder der Anwendungsbereich des vorliegenden Dokuments berührt wird. Daher soll die vorliegende Erfindung nicht auf die hierin gezeigten Ausführungsformen beschränkt werden, sondern es soll ihr der größtmögliche Anwendungsbereich eingeräumt werden, der mit den hierin offenbarten Grundsätzen und neuen Merkmalen vereinbar ist.

## Patentansprüche

1. Medizinisches System (100; 200) zur Unterstützung bei einer Ablationstherapie in einem Ablationsbereich (A), umfassend:
mindestens eine nagelförmige Antenne (10; 10') mit einem entlang einer Längsachse bewegbaren leitenden Element (11), welche dazu eingerichtet ist, elektromagnetische Strahlungen in Mikrowellen- oder Radiofrequenzen abzustrahlen;
eine Temperaturmessungseinheit (20), welche dazu ausgelegt ist, eine Temperatur zu messen und entsprechende Signale auszugeben;
eine Simulationseinheit (30), welche dazu eingerichtet ist, basierend auf dem Standort der mindestens einen Antenne (10, 10') und der abgestrahlten elektromagnetischen Strahlungen und mit Hilfe eines Wärmeausbreitungsmodells, eine Simulation der Temperaturverteilung in dem Ablationsbereich (A) bereitzustellen;
eine Konfigurationseinheit (40), welche dazu eingerichtet ist, basierend auf den Signalen der Temperaturmessungseinheit (20) und der bereitgestellten Simulation der Temperaturverteilung, eine Betriebskonfiguration der mindestens einen Antenne (10, 10') zu ermitteln, wobei die ermittelte Betriebskonfiguration mindestens eine Bewegung des leitenden Elements (11) entlang der Längsachse der nagelförmigen Antenne (10, 10') umfasst; und
eine Ausgabeeinheit (50), welche dazu eingerichtet ist, ein der ermittelten Betriebskonfiguration entsprechenden Konfigurationssignal (K) auszugeben.

2. System (100; 200) nach Anspruch 1, wobei die Betriebskonfiguration ferner umfasst: eine Umstellung der Leistung der elektromagnetischen Strahlungen; und/oder eine Umstellung der Frequenz der elektromagnetischen Strahlungen; und/oder eine Umstellung der Phase der elektromagnetischen Strahlungen.

3. System (100; 200) nach einem der vorangehenden Ansprüche mit mindestens zwei Antennen (10, 10'), wobei die erste Antenne (10; 10') mit einem ersten elektromagnetischen Feld und die zweite Antenne (10'; 10) mit einem zweiten elektromagnetischen Feld versorgt wird, wobei das erste Feld als Hintergrundfeld dient.

4. System (100; 200) nach einem der vorangehenden Ansprüche mit mindestens zwei Antennen (10, 10'), wobei die jeweiligen Phasen der abgestrahlten elektromagnetischen Strahlungen umstellbar sind.

5. System (100; 200) nach einem der vorangehenden Ansprüche mit mindestens zwei Antennen (10, 10'), wobei die Antennen (10, 10') innerhalb einer Führungsrohr-Längenhaltestruktur (13) schirmförmig angeordnet sind, und wobei die Antennen (10, 10') elektromagnetische Strahlungen unterschiedlicher Eigenschaften abstrahlen.

6. System (100; 200) nach einem der vorangehenden Ansprüche, wobei die Konfigurationssignale (K) in einem zeitlichen Abstand von zumindest fünf Sekunden zueinander, vorzugsweise im Sekundentakt, ausgegeben werden.

7. System (100; 200) nach einem der vorangehenden Ansprüche, wobei die Temperaturmessungseinheit (20) eine Vielzahl von Temperatursensoren (201, 202) umfasst, welche in die mindestens eine Antenne (10, 10') integriert sind und/oder sich als gesonderten Messsonden auf dem Ablationsbereich (A) befinden.

8. Computerimplementiertes Verfahren zur Unterstützung bei einer Ablationstherapie in einem Ablationsbereich (A), vorzugsweise zur Verwendung mit dem System (100) nach Ansprüche 1 bis 7, mit den Schritten:
Bereitstellen (S1), basierend auf dem Standort der mindestens einen Antenne (10, 10') und der abgestrahlten elektromagnetischen Strahlungen und mit Hilfe eines Wärmeausbreitungsmodells, einer Simulation der Temperaturverteilung in dem Ablationsbereich (A);
Messen (S2) einer Temperatur mit Hilfe einer Temperaturmessungseinheit (20);
Ermitteln (S3), basierend auf der ermittelten Temperatur und der bereitgestellten Simulation der Temperaturverteilung in dem Ablationsbereich (A), einer Betriebskonfiguration der mindestens einen Antenne (10, 10'), wobei die ermittelte Betriebskonfiguration mindestens eine Bewegung des leitenden Elements (11) entlang der Längsachse der nagelförmigen Antenne (10, 10') umfasst; und
Ausgeben (S4) eines der ermittelten Betriebskonfiguration entsprechenden Konfigurationssignals (K).

9. Computerprogrammprodukt (300), umfassend einen ausführbaren Programmcode (350), der dazu ausgelegt ist, wenn er ausgeführt wird, das computerimplementierte Verfahren gemäß Patentanspruch 8 durchzuführen.

10. Nicht-flüchtiges computerlesbares Datenspeichermedium (400), umfassend einen ausführbaren Programmcode (450), der dazu ausgelegt ist, wenn er ausgeführt wird, das computerimplementierte Verfahren gemäß Patentanspruch 8 durchzuführen.
